# FASCICULE DE BREVET EUROPEEN

(11) **EP 4 212 877 B1**
(45) Date de publication et mention de la délivrance du brevet: **19.03.2025**
(21) Numéro de dépôt: 23156126.7
(22) Date de dépôt: 19.05.2020
(51) Int. Cl.: G01N 33/564

(54) **UTILISATION DES PROTÉINES PD-1 ET CD38 COMME MARQUEURS D'UNE PATHOLOGIE AUTO-IMMUNE ACTIVE**
VERWENDUNG VON PD-1 UND CD38 PROTEINEN ALS MARKER FÜR EINE AKTIVE AUTOIMMUNERKRANKUNG
USE OF PD-1 AND CD38 PROTEINS AS MARKERS OF ACTIVE AUTOIMMUNE DISEASE

(30) Priorité: 22.05.2019 FR 1905358
(43) Date de publication de la demande: 19.07.2023
(62) Demande divisionnaire de: 20726132.2
(73) Titulaire: Nantes Université, 44035 Nantes Cedex 1 (FR); Centre Hospitalier Universitaire De Nantes, 44000 Nantes (FR); Institut National de la Santé et de la Recherche Médicale (INSERM), 75013 Paris (FR)
(72) Inventeur: RENAND, Amédée, 44980 SAINTE-LUCE-SUR-LOIRE (FR); CONCHON, Sophie, 44300 NANTES (FR); GOURNAY, Jérôme, 44100 NANTES (FR); AUBLE, Hélène, 44000 NANTES (FR); HABES, Sarah, 44200 NANTES (FR)
(74) Mandataire: Novagraaf Technologies

(56) Documents cités:
- WO-A2-2006/096800
- THOMAS VOLLBRECHT ET AL: "Impact of changes in antigen level on CD38/PD-1 co-expression on HIV-specific CD8 T cells in chronic, untreated HIV-1 infection", JOURNAL OF MEDICAL VIROLOGY, vol. 82, no. 3, 1 March 2010 (2010-03-01), US, pages 358 - 370, XP055671471, ISSN: 0146-6615, DOI: 10.1002/jmv.21723
- LIANG MA ET AL: "Tfh and plasma cells are correlated with hypergammaglobulinaemia in patients with autoimmune hepatitis", LIVER INTERNATIONAL, vol. 34, no. 3, 5 July 2013 (2013-07-05), GB, pages 405 - 415, XP055671580, ISSN: 1478-3223, DOI: 10.1111/liv.12245
- AMÉDÉE RENAND ET AL: "Integrative molecular profiling of autoreactive CD4 T cells in autoimmune hepatitis", BIORXIV, 7 January 2020 (2020-01-07), XP055671453, Retrieved from the Internet <URL:https://www.biorxiv.org/content/10.1101/2020.01.06.895938v1.full.pdf> [retrieved on 20200225], DOI: 10.1101/2020.01.06.895938
- NARUHIRO KIMURA ET AL: "Possible involvement of chemokine C-C receptor 7 - programmed cell death-1 + follicular helper T-cell subset in the pathogenesis of autoimmune hepatitis : Follicular helper T cell subsets in AIH", JOURNAL OF GASTROENTEROLOGY AND HEPATOLOGY, vol. 33, no. 1, 28 December 2017 (2017-12-28), pages 298 - 306, XP055671735, ISSN: 0815-9319, DOI: 10.1111/jgh.13844
- GUTKOWSKI K ET AL: "Su.112. Expression of Cd38 and Cd5 Surface Markers On Peripheral Blood B Lymphocytes (Cd19+) in Patients with Autoimmune Hepatitis; Influence of Prednisone and Azathioprine Five Months Therapy", CLINICAL IMMUNOLOGY, ACADEMIC PRESS, US, vol. 119, 1 January 2006 (2006-01-01), pages S198, XP024911718, ISSN: 1521-6616, [retrieved on 20060101], DOI: 10.1016/J.CLIM.2006.04.539

## Description

### Domaine technique

La présente invention se rapporte à un procédé de prédiction et/ou de pronostic d'une hépatite auto-immune active chez un sujet, comprenant la mesure des quantités de certaines protéines dans un échantillon biologique du sujet, ainsi qu'à certaines utilisations de ces protéines.

Dans la description ci-dessous, les références entre crochets ([ ]) renvoient à la liste des références présentée à la fin du texte.

### Etat de la technique

L'hépatite auto-immune (HAI) est une maladie chronique du foie dont les mécanismes immunologiques restent inconnus. C'est une maladie rare avec une incidence de 1.1 à 1.9 cas par 100000 personnes par an en Europe, caractérisée par une attaque du parenchyme hépatique par les cellules du système immunitaire de type lymphoïde (Liberal et al. : Cutting edge issues in autoimmune hepatitis. J. Autoimmun. (2016) 75, 6-19 ([1])).

Le diagnostic d'une HAI est souvent long à être posé. Il repose, comme pour les autres maladies auto-immunes du foie, sur l'élimination d'autres causes, sur des tests biologiques et biochimiques sanguins peu spécifiques (élévation des transaminases, absence d'hépatite virale, présence d'auto-anticorps et une hyper gammaglobulinémie) et sur la lecture histologique de biopsie hépatique. Dans les autres cas de désordre auto-immun hépatique ou non, le diagnostic est basé sur les symptômes, la présence ou non d'auto-anticorps et sur la lecture histologique du tissue touché. Le suivi de la réponse aux traitements est généralement basé sur l'amélioration des symptômes, la régulation des paramètres biologiques et éventuellement sur la lecture histologique de biopsie des tissues touchés.

Le traitement associant corticoïdes et azathioprine, inchangé depuis plus de 30 ans (Manns et al. : Diagnosis and management of autoimmune hepatitis. Hepatol. Baltim. Md 51, 2010, 2193-2213 ([2])), vise à contrôler l'inflammation pour éviter la progression vers la fibrose hépatique. Cette immunosuppression non sélective est suffisante pour induire une rémission complète chez 70% des patients la première année. Les recommandations européennes préconisent de tenter d'arrêter l'azathioprine après 2 ans de rémission complète pour limiter les effets néfastes de ceci à long terme (European Association for the Study of the Liver (2015). EASL Clinical Practice Guidelines: Autoimmune hepatitis. J. Hepatol. 63, 971-1004 ([3])) ; toutefois le risque de rechute est élevé et concerne 60 à 80 % des patients dans les six à douze mois suivant l'arrêt du traitement (Manns et al., 2010 ([2])). L'état de rémission clinique complète repose actuellement sur des tests biologiques et biochimiques, notamment par une normalisation des transaminases et des IgG sanguins, qui ne prédisent pas le risque de rechute.

Une meilleure compréhension/caractérisation de la réponse immune dans l'HAI serait utile pour prédire le risque de rechute, mais aussi pour identifier les protagonistes immunologiques impliqués dans la pathogénèse de cette maladie.

Les lymphocytes T CD4 (LT CD4) et les LT CD8 ont été décrits comme fortement impliqués dans l'attaque du parenchyme hépatique dans l'HAI. Ils sont largement représentés au niveau de l'infiltrat inflammatoire portal et intra lobulaire. Toutefois, la relation entre les acteurs immunologiques impliqués et l'évolution de la maladie n'est pas encore connue (Liberal et al., 2016 ([1])).

Récemment, ont été mises en évidence des altérations lymphocytaires sanguines au diagnostic, qui persistent lors de la phase de rémission sous traitement (Renand et al., : Immune Alterations in Patients With Type 1 Autoimmune Hepatitis Persist Upon Standard Immunosuppressive Treatment. Hepatol. Commun. (2018) 2, 968-981 ([4])). Cependant certaines de ces altérations lymphocytaires étaient retrouvées chez des patients atteints de stéatohépatite dysmétabolique (NASH), et semblaient donc refléter l'inflammation hépatique non spécifique (Böttcher et al. : MAIT cells are chronically activated in patients with autoimmune liver disease and promote pro-fibrogenic hepatic stellate cell activation. Hepatol. Baltim. Md, 2018 ([5]); Jeffery et al. : Biliary epithelium and liver B cells exposed to bacteria activate intrahepatic MAIT cells through MRI. J. Hepatol., 2016, 64, 1118-1127 ([6]); Oo et al.: CXCR3-dependent recruitment and CCR6-mediated positioning of Th-17 cells in the inflamed liver. J. Hepatol. 57, 1044-1051, 2012 ([7])). Par ailleurs, des cas d'inflammation hépatique non virale (comme la NASH) avec des signes d'auto-immunité (présence d'auto- anticorps sanguins chez 10% des patients NASH) sont répertoriés, mais ne font pour l'instant l'objet d'aucun traitement spécifique.

WO2006/096800 décrit un procédé de diagnostic et de surveillance de la progression en réponse à la thérapie d'un sujet atteint d'hépatite auto-immune. Dans ce procédé sont quantifiées les cellules T réactives avec des peptides immunogènes comprenant des épitopes de lymphocytes T impliqués dans l'hépatite auto-immne.

Ainsi, il existe un réel besoin d'identifier une signature spécifique de l'auto-immunité hépatique, notamment pour en améliorer le diagnostic ou la prise en charge clinique et thérapeutique.

### Description de l'invention

Au terme d'importantes recherches, les inventeurs sont parvenus à résoudre ce problème technique.

De manière surprenante, les inventeurs ont en effet mis en évidence une signature spécifique de l'atteinte auto-immune hépatique.

Les inventeurs ont en effet démontré, par cytométrie en flux sur des lymphocytes T (CD3+) sanguins, que la détection des marqueurs PD-1 (Programmed cell death 1) et CD38, éventuellement en association avec les marqueurs CD3, CD4, CD8, CD45RA, CXCR5, CD127 et CD27, met en évidence une signature auto-immune hépatique. Cette signature auto-immune permet avantageusement de discriminer les patients ayant une hépatite auto-immune active (HAI) des patients ayant une inflammation hépatique non-auto-immune (NASH).

Les avantages de l'invention sont nombreux.

Par exemple, l'invention peut permettre de réduire le temps de diagnostic en apportant de nouvelles informations complémentaires à celles classiquement obtenues.

L'invention a également pour avantage de fournir un moyen permettant une meilleure orientation et/ou une confirmation du diagnostic, notamment dans des cas atypiques (forme séronégative, sans autoanticorps) d'HAI.

L'invention peut en outre permettre d'évaluer l'activité auto-immune chez les patients. Une meilleure prise en charge des patients peut donc être avantageusement envisagée, avec par exemple un aménagement ou non du traitement proposé.

Avantageusement, l'invention est applicable aux autres désordres auto-immuns hépatiques et non-hépatiques qui sont confrontés à la même problématique.

En outre, l'invention permet avantageusement de mettre en évidence parmi des patients ayant une inflammation hépatique un sous-groupe de patients ayant une auto-immunité hépatique associée. Une amélioration de leur prise en charge clinique et thérapeutique est donc avantageusement envisagée.

Avantageusement, l'invention permet une évaluation simplifiée de l'activité auto-immune chez un patient, puisqu'elle peut être réalisée par la détection d'une signature auto-immune selon l'invention dans le sang du patient, par exemple au moyen d'une simple prise de sang.

L'invention permet en outre de manière avantageuse de mesurer dans le sang l'activité des lymphocytes T qui sont les acteurs principaux de l'auto-immunité. Cette méthode est moins invasive pour le patient qu'une biopsie, et permet d'apporter une plus-value aux tests biologiques déjà connus. En outre, le fait de mesurer directement l'activité des protagonistes de la pathologie dans le sang apporte, de manière avantageuse, une meilleure sensibilité et éventuellement un complément dans le cas des diagnostic et suivi atypiques, comme par exemple dans les formes séronégatives ou à faible infiltrat lymphocytaire du tissu.

Ainsi, un premier objet de l'invention se rapporte à un procédé de prédiction et/ou de pronostic d'une pathologie auto-immune active chez un sujet, comprenant la détection de la co-expression des protéines PD-1 et CD38 à la surface des lymphocytes T dans un échantillon biologique du sujet, selon les revendications 1-9 annexées.

Selon l'invention, la pathologie auto-immune active est une hépatite auto-immune. Par exemple, le sujet chez qui la prédiction et/ou le pronostic est recherché peut être un patient ayant une inflammation hépatique.

On entend par « diagnostic », la mise en évidence d'une auto-immunité chez des patients. Cette mise en évidence peut avantageusement permettre d'en déduire ou de confirmer une atteinte du patient par une HAI, ou de mettre en évidence une atteinte auto-immune dans le cadre d'une pathologie à dominante non-auto-immune. Par exemple, le diagnostic peut être réalisé sur des formes atypiques d'HAI, par exemple une forme séronégative, c'est-à-dire sans autoanticorps, et/ou avec peu ou pas d'infiltrat lymphocytaire du tissu hépatique. Dans un autre exemple, le diagnostic peut être une discrimination des patients ayant une hépatite auto-immune des patients ayant une stéatose hépatique non-alcoolique (NASH), ou encore une mise en évidence d'une auto-immunité hépatique chez les patients atteints de NASH.

On entend par « prédiction », au sens de la présente invention, l'anticipation de la progression de la maladie chez un sujet lors d'un suivi au cours du temps. Il peut s'agir notamment d'une prédiction d'un risque, par exemple du risque de développer une pathologie auto-immune chez un sujet, ou du risque de rechute d'un patient atteint d'une pathologie auto-immune notamment suite à l'arrêt ou à l'aménagement de son traitement, ou d'un évènement indésirable immun (immun related adverses events : Iraes) par exemple lors de l'utilisation d'anticorps ciblant des checkpoint inhibitors dans le cadre d'une immunothérapie anti-cancer.

On entend par « pronostic », au sens de la présente invention, la prédiction des chances de survie d'un patient atteint d'une pathologie auto-immune active chez un sujet. Par exemple, la méthode de pronostic de l'invention peut permettre d'évaluer le taux de survie, ce taux indiquant le pourcentage de personnes ayant participé à une étude et vivant pendant une période donnée, généralement 5 à 10 ans après le diagnostic d'une pathologie auto-immune active. Généralement, un bon pronostic, au sens de l'invention, est associé à une survie médiane supérieure à 5 ans, alors qu'un mauvais pronostic est associé à une survie médiane inférieure à 5 ans.

On entend par « détection de la co-expression des protéines », au sens de la présente invention, toute détection de l'expression conjointe des protéines PD-1 et CD38 à la surface de chaque lymphocyte T présent l'échantillon biologique. C'est donc la fréquence d'expression conjointe des protéines PD-1 et CD38 à la surface de chaque lymphocyte T présent dans l'échantillon biologique qui est mesurée. La détection peut être par exemple une comparaison entre le niveau de la fréquence d'expression conjointe de ces protéines dans un échantillon biologique d'un sujet, et un échantillon ou une valeur de référence (encore appelée « valeur seuil » ou « valeur normale ») permettant un diagnostic, une prédiction et/ou un pronostic avéré de la pathologie auto-immune active. En effet, la valeur de référence représente la fréquence d'expression conjointe de ces protéines dans la population ne présentant pas la pathologie recherchée. La valeur de référence peut être définie sur la base de mesures obtenues sur un échantillon de sujets sains, c'est-à-dire ne présentant pas la pathologie recherchée. Il peut s'agir par exemple de sujets qui n'ont pas d'hépatite virale, ni de signe d'auto-immunité au niveau du foie et/ou d'affection chronique hormis, éventuellement, une inflammation hépatique due à un dérèglement dysmétabolique (également appelé « Foie gras » ou « maladie du soda »). Ainsi, on comprend que les « sujets sains » permettant de définir la valeur de référence peuvent être des patients atteints de NASH (ou non atteints de NASH), mais ne présentant pas d'hépatite virale, ni de signe d'auto-immunité au niveau du foie et/ou d'autre affection chronique hormis l'inflammation hépatique due à un dérèglement dysmétabolique. La valeur de référence peut être définie par exemple selon le protocole décrit dans l'exemple 2 ci-après. La valeur de référence peut être établie sur un nombre significatif de sujets sains, par exemple au moins100 sujets sains. Avantageusement, la valeur seuil peut être définie comme supérieure à une fréquence de LTCD3+ exprimant conjointement PD1 et CD38 de 2,77 pourcent. Ainsi, une fréquence supérieure à 2,77 pourcent sera caractéristique d'une auto-immunité hépatique. La détection peut être réalisée par toute méthode connue de l'homme du métier. Il peut s'agir par exemple d'une méthode choisie parmi la cytométrie en flux ou de masse, une technologie de test immunologique, telle que ELISA direct, ELISA indirect, ELISA sandwich, ELISA compétitif, ELISA multiplex, un dosage radio-immunologique (RIA) ou une technologie ELISPOT, une méthode d'analyse par spectrométrie de masse, un procédé de chromatographie, un procédé de qPCR et une combinaison d'au moins deux de ces méthodes. Ainsi, dans un exemple décrit, la détection d'une signature auto-immune peut être réalisée dans le sang, par exemple du sang total, du patient par une simple prise de sang suivi d'un test simplifié consistant à incuber les cellules sanguines avec un mélange d'anticorps spécifiques fluorescents, suivi d'une analyse sur un cytomètre en flux.

Les protéines PD-1 et CD38 peuvent être détectées à la surface de toute cellule connue pour les exprimer à leur surface. Il peut s'agir par exemple des lymphocytes T CD3 et/ou T CD8 et/ou lymphocytes T CD4.

L'échantillon biologique utilisé peut être tout échantillon biologique communément utilisé pour des analyses biologiques. Il peut s'agir par exemple d'un échantillon de sang, par exemple une fraction du sang, ou alternativement un échantillon de sang total, ou d'une fraction de biopsie, par exemple hépatique, de tissu. Dans le procédé de l'invention, l'échantillon biologique est un échantillon de sang.

Le procédé de l'invention comprend les étapes consistant à :
(i) détecter la co-expression de PD-1 et CD38, à la surface des lymphocytes T, dans ledit échantillon de sang et déterminer la fréquence de co-expression de PD-1 et CD38 ;
(ii) comparer la fréquence de co-expression de PD-1 et CD38 déterminée en (i) avec une valeur de référence de la fréquence de co-expression de PD-1 et de CD38,
(iii) rechercher la présence ou l'absence d'un écart de la fréquence de co-expression de PD-1 et de CD38 déterminée en (ii) par rapport à la valeur de référence ;
(iv) attribuer la présence ou l'absence d'écart à une prédiction et/ou un pronostic de l'hépatite auto-immune active chez le sujet.

Une fréquence de co-expression de PD-1 et CD38, dans ledit échantillon, supérieure à une valeur de référence indique que le sujet présente un risque de rechute de la pathologie auto-immune active.

Outre les protéines PD-1 et CD38, il est possible, selon l'invention, de détecter la co-expression de PD-1 et de CD38 avec au moins un autre marqueur biologique choisi dans le groupe consistant en CD3, CD4, CD8, CD45RA, CXCR5, CD127 et CD27. Il peut s'agir d'un marqueur, ou de deux marqueurs, ou de trois marqueurs, ou de quatre marqueurs, ou de cinq marqueurs, ou de six marqueurs, ou de sept marqueurs. Cette détection d'au moins un autre marqueur peut être réalisée en même temps que la détection de la co-expression de PD-1 et CD38, ou bien ultérieurement à l'étape de détection de la co-expression de PD-1 et CD38. Par exemple, on peut détecter la co-expression des associations de marqueurs biologiques choisis parmi les associations PD-1/CD38/CD3, PD-1/CD38/CD3/CD4/CD8/CD45RA, PD-1/CD38/CD3/CD4/CD8/CD45RA/CD127, PD-1/CD38/CD3/CD4/CD8/CD45RA/CXCR5/CD127 et PD-1/CD38/CD3/CD4/CD8/CD45RA/CXCR5/CD127/CD27. Avantageusement, l'analyse de la co-expression de PD-1 et de CD38 à la surface des LT CD3 (CD3+) peut être réalisée en association avec la détection de la fréquence des populations lymphocytaires définis par les phénotypes ci-dessous:
a) CD45RA (étude de la sous-population CD3+CD45RA-PD-1+CD38+) ;
b) CD45RA, CD4 et CD8 (étude des sous-populations CD3+CD4+CD8-CD45RA-PD-1+CD38+ et CD3+CD4-CD8+CD45RA-PD-1+CD38+) ;
c) CD45RA, CD4, CD8 et CD127 (étude des sous-populations CD3+CD4+CD8-CD45RA-CD127-PD-1+CD38+ et CD3+CD4-CD8+CD45RA-CD127-PD-1+CD38+) ;
d) CD45RA, CD4, CD8, CD127 et CXCR5 (étude des sous-populations CD3+CD4+CD8-CD45RA-CD127-CXCR5-PD-1+CD38+ et CD3+CD4-CD8+CD45RA-CD127- CXCR5-PD-1+CD38+) ; et
e) CD45RA, CD4, CD8, CD127, CXCR5 et CD27 (étude des sous-populations CD3+CD4+CD8-CD45RA-CD127-CXCR5-CD27+PD-1+CD38+ et CD3+CD4-CD8+CD45RA-CD127- CXCR5-CD27+PD-1+CD38+).

Avantageusement, les associations des marqueurs cités peuvent permettent de mettre en évidence des populations lymphocytaires T CD4 et CD8 qui sont significativement augmentées chez les patients ayant une pathologie auto-immune active, notamment une HAI active, en comparaison à une valeur de référence, ou par exemple aux patients atteints de NASH lorsqu'il s'agit d'une comparaison avec la HAI.

Un autre objet de l'invention se rapporte à l'utilisation *in vitro* du pool de protéines PD-1 (Programmed cell death 1) et CD38 comme marqueurs biologiques co-exprimés à la surface des lymphocytes T, mesurés dans un échantillon de sang prélevé sur un sujet pour la prédiction et/ou le pronostic d'une hépatite auto-immune active chez un sujet.

Un autre objet décrit (non revendiqué) se rapporte à un dispositif pour détecter la co-expression de PD-1 et de CD38 à la surface des lymphocytes T dans un échantillon prélevé sur un sujet, comprenant :
(i) éventuellement des moyens pour obtenir un échantillon prélevé sur le sujet, et
(ii) des moyens pour détecter la co-expression de PD-1 et de CD38 à la surface des lymphocytes T dans ledit échantillon, et
(iii) des moyens de détermination de la fréquence de co-expression de PD-1 et de CD38 dans l'échantillon. Avantageusement, lesdits moyens de détermination de la fréquence de co-expression peuvent permettre d'indiquer si cette fréquence, dans ledit échantillon, est supérieure ou inférieure à une valeur de référence et/ou si cette fréquence dans ledit échantillon diffère ou non d'une valeur de référence, ladite valeur de référence représentant un diagnostic, une prédiction et/ou un pronostic d'une pathologie auto-immune active chez le sujet.

D'autres avantages pourront encore apparaître à l'homme du métier à la lecture des exemples ci-dessous, illustrés par les figures annexées, donnés à titre illustratif.

### Brève description des figures

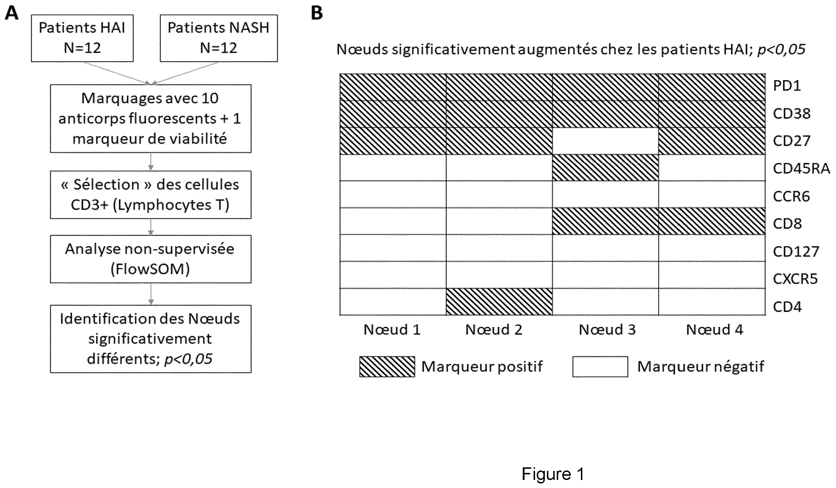

[Fig. 1] représente : A) représentation de la stratégie d'analyse non supervisée (FlowSOM) des données de cytométrie en flux chez 12 patients HAI et 12 patients NASH. B) Heatmap représentant l'expression des marqueurs de surface des 4 populations lymphocytaires (nœuds) présentants une augmentation significative (p<0.05)dans l'HAI. Intensité d'expression allant du rayée (forte expression) au blanc (faible expression).

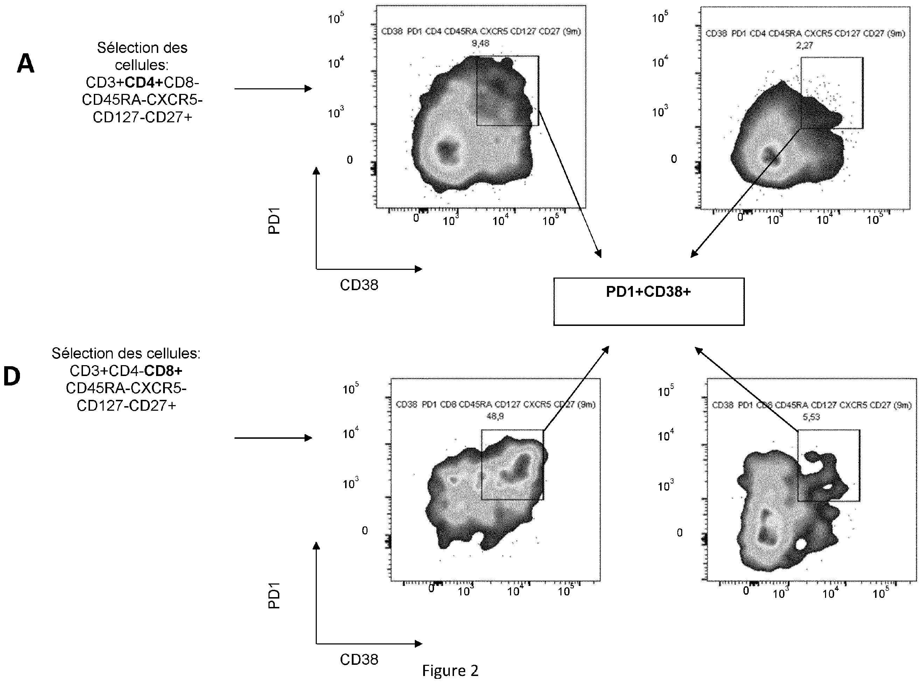

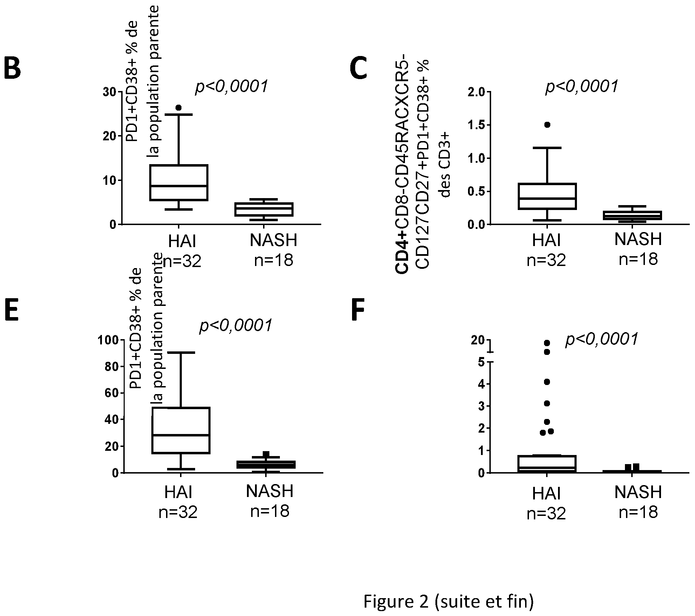

[Fig. 2] représente : A et D) Représentation des données de cytométrie en flux des marqueurs PD-1 et CD38 sur les populations CD3+CD4+CD45RA-CXCR5-CD127-CD27+ (A) et CD3+CD8+CD45RA-CXCR5-CD127-CD27+ (D). B) et E) graphiques représentant le pourcentage des cellules PD-1+CD38+ par cellules CD3+CD4+CD45RA·CXCR5-CD127-CD27+ (B) et par cellules CD3+CD8+CD45RA-CXCR5-CD127-CD27+ (E) chez 32 patients HAI et 18 patients NASH. C et F) graphiques représentant le pourcentage des cellules CD4+CD45RACXCR5-CD127-CD27+PD-1 +CD38+ (C) et CD8+CD45RA-CXCR5-CD127-CD27+PD1+CD38+ (F) par cellules CD3+. Les p value ont été déterminées à l'aide du test statistique Mann-Whitney.

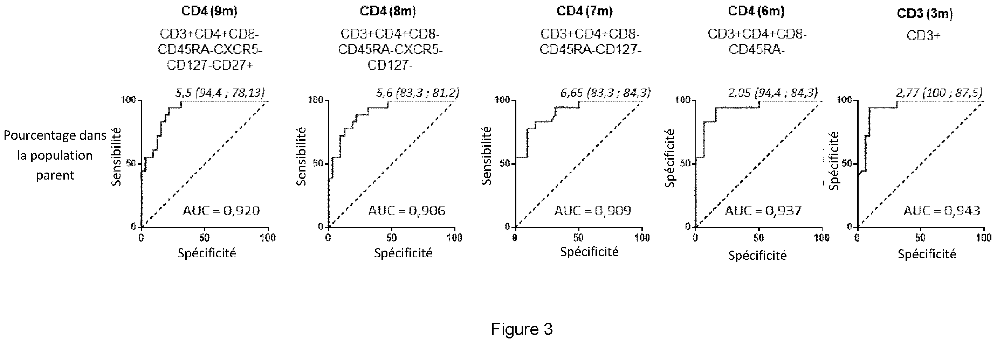

[Fig. 3] représente les courbes ROC basées sur le pourcentage de cellules PD-1+CD38+ des LTCD4 et des LTCD3 dans la population parent identifiée avec les marqueurs annotés au-dessus de la courbe ROC. En italique la valeur seuil est indiquée en noir avec entre parenthèses les valeurs de sensibilité et de spécificité. Les valeurs des AUC sont indiquées sur chaque graphique.

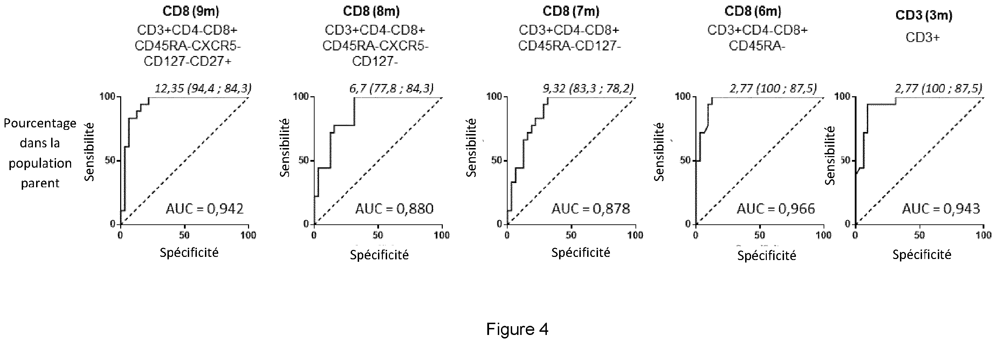

[Fig. 4] représente les courbes ROC basées sur le pourcentage de cellules PD-1+CD38+ des LTCD8 et des LTCD3 dans la population parent identifiée avec les marqueurs annotés au-dessus de la courbe ROC. En italique la valeur seuil est indiquée en noir avec entre parenthèse les valeurs de sensibilité et de spécificité. Les valeurs des AUC sont indiquées sur chaque graphique.

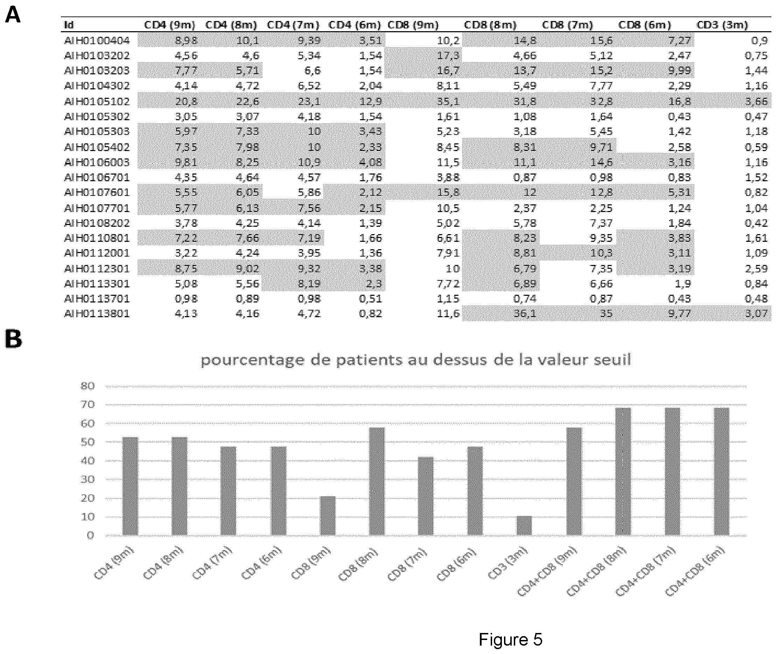

[Fig. 5] est une représentation des valeurs des données de cytométrie en flux des marqueurs PD-1 et CD38 chez des patients HAI en rémission complète sous traitement. A) Heatmap représentant des valeurs des données de cytométrie en flux des marqueurs PD-1 et CD38 chez des patients HAI en rémission complète sous traitement En gris les données supérieures aux valeurs seuils identifiées précédemment. B) graphiques représentant le pourcentage de patients en rémission complète sous traitement ayant des valeurs des marqueurs PD-1 et CD38 au-dessus des valeurs seuils déterminées précédemment (figures 3 et 4).

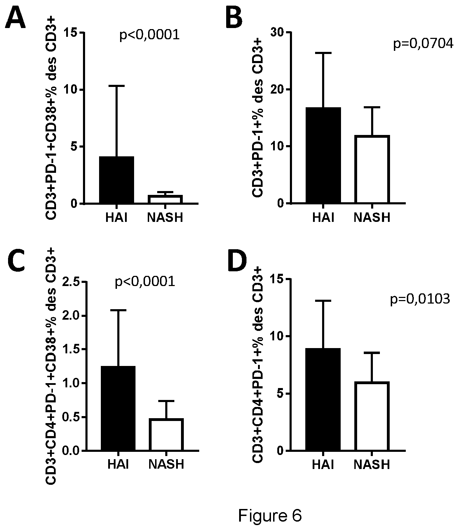

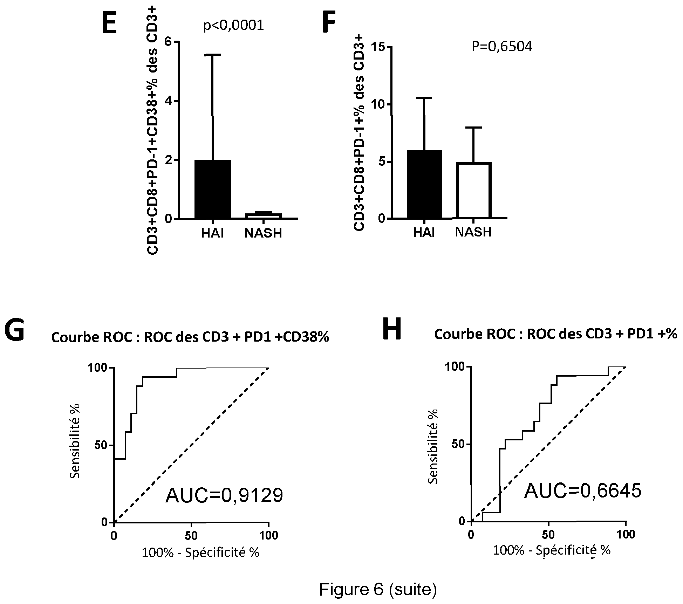

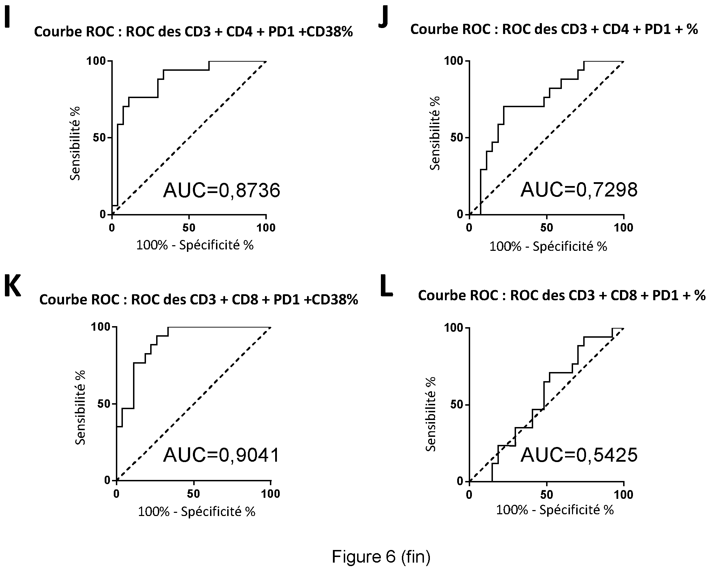

[Fig.6] est une représentation des données d'analyse entre la mesure simple de PD-1 à la surface des lymphocytes T versus la mesure de la co-expression de PD-1 avec CD38 à la surface des lymphocytes T. Comparaison du pourcentage de cellules CD3+PD-1+CD38+ (A), CD3+PD-1+ (B), CD3+CD4+PD-1+CD38+ (C), CD3+CD4+PD-1+ (D), CD3+CD8+PD-1+CD38+ (E) et CD3+CD8+PD-1+ (F) des CD3+ entre des patients atteints de HAI et des patients atteints de NASH. Les p value ont été déterminées à l'aide du test statistique Mann-Whitney. Les courbes ROC (sensibilité/spécificité en %) basées sur le pourcentage de cellules CD3+PD-1+CD38+ (G), CD3+PD-1+ (H), CD3+CD4+PD-1+CD38+ (I), CD3+CD4+PD-1+ (J), CD3+CD8+PD-1+CD38+ (K) et CD3+CD8+PD-1+ (L) des CD3+ entre des patients atteints de HAI et des patients atteints de NASH sont représentées. Les valeurs des AUC sont indiquées sur chaque graphique.

### Exemples

### Exemple 1 : Identification des populations lymphocytaires différentielles entre des patients atteints de HAI et des patients atteints de NASH

Dans l'optique d'identifier une signature spécifique de l'auto-immunité hépatique, nous avons réalisé des analyses de données de cytométrie en flux multiparamétrique par des méthodes statistiques non-supervisées (FlowSOM) entre 12 patients HAI et 12 patients NASH, qui représentent le meilleur choix comme population contrôle pour répondre à notre question.

Cette approche non supervisée a permis d'établir une stratégie d'identification par cytométrie en flux, en utilisant un total de 13 paramètres dont 10 marqueurs de surface cellulaire (CD3, CD4, CD8, CD45RA, CXCR5, CCR6, CD27, CD127, PD-1 et CD38) et 1 marqueur de viabilité, de deux populations cellulaires (LT CD4 et CD8) qui se caractérisent par l'expression du PD-1 et du CD38 (Figure 1).

Sur une cohorte de 32 patients HAI actifs et de 18 patients NASH, les deux populations cellulaires LT CD4 et LT CD8 exprimant PD-1 et CD38 sont significativement augmentées dans le sang des patients HAI en comparaison aux patients NASH (Figure 2). Cette stratégie permet de facilement discriminer auto-immunité hépatique (HAI) et inflammation hépatique (courbe ROC; Figures 3 et 4).

En comparaison la mesure seule de l'expression de PD-1 à la surface des lymphocytes T (CD3+, CD3+CD4+ et CD3+CD8+) est beaucoup moins sensible pour discriminer auto-immunité hépatique (HAI) et inflammation hépatique (NASH) que la mesure de la co-expression de PD-1 et CD38 à la surface des lymphocytes T (Figure 6. Courbe ROC). En effet, l'analyse de la différence de fréquence de lymphocytes T exprimant PD-1 entre patients HAI actifs et patients NASH est beaucoup moins significative que les résultats obtenus avec la mesure de la co-expression de PD-1 et de CD38 (Figure 6).

Ces résultats démontrent l'intérêt de l'utilisation de ces marqueurs, notamment en cytométrie en flux, comme potentiels biomarqueurs de l'auto-immunité hépatique.

### Exemple 2 : Détermination de valeurs seuils des biomarqueurs

Dans le but de proposer des valeurs seuils les plus sensibles possibles, nous avons analysés de diverses manières les résultats générés (Figures 3 et 4). Le principe a été de réaliser l'analyse du pourcentage d'expression de PD-1 et du CD38 en modifiant le nombre de marqueurs associés, notamment en vue d'une simplification d'utilisation ultérieure.

Sur les population LT CD4 (Figure 3) et LT CD8 (Figure 4), et sur la population LT CD3 total (Figures 3 et 4), le pourcentage d'expression de PD-1 et du CD38 (dans la population cellulaire parente selon une stratégie de « gating » pour l'analyse des résultats de cytométrie en flux) a été analysé dans les sous populations CD45RA- (6m); CD45RA-CD127- (7m); CD45RA-CD127-CXCR5-(8m) et CD45RA-CD127-CXCR5-CD27+ (9m) (Figures 3 et 4). L'analyse a été réalisée à l'aide des courbes ROC. Dans toutes les conditions d'analyse nous obtenons des aires sous la courbe (AUC) supérieures à 0,85, ce qui démontre le fort potentiel de ces marqueurs comme biomarqueurs de l'auto-immunité hépatique.

Des valeurs seuils de la signature auto-immune ont ainsi pu être définies avec des sensibilités allant de 77% à 100% et des spécificités allant de 78% à 87%.

Sur un petit groupe de patients HAI en rémission complète sous traitement (étude pilote) nous avons observé qu'environ 50% des patients HAI en rémission complète sous traitement (n=19) maintiennent un taux élevé de ces biomarqueurs, alors qu'il est normalisé chez les autres (Figure 5). Ceci suggère une possible association entre la persistance de ces biomarqueurs sanguins et le devenir du patient (risque de rechute).

L'ensemble de ces résultats démontre qu'il est possible de définir une signature lymphocytaire auto-immune dans le sang des patients qui est discriminante de l'inflammation non-spécifique.

### Exemple 3 : Recherche de combinaisons de biomarqueurs d'intérêt

L'étude porte sur 1) l'interprétation clinique des données de cytométrie; 2) la validation des données sur un plus grand nombre de patients et dans d'autres pathologies auto-immune; et 3) sur la simplification et le développement d'un prototype de test sur sang total.

Concernant la simplification et le développement d'un prototype de test sur sang total, différentes associations de regroupement des marqueurs précédemment cités sont testées, afin de regrouper les marqueurs négatifs ensemble pour créer des «fenêtres» d'exclusions et ainsi faciliter la lecture du test.

Par exemple, pour le panel 9m, une première sélection des cellules T CD3+CD45RA-CXCR5-CD127- est effectuée, CD45RA, CXCR5 et CD127 étant regroupés dans un même canal de lecture pour faciliter leurs exclusions. Il est ainsi possible de passer de 9 canaux de lecture en cytométrie en flux à 6 ou 7 canaux.

Ceci permet par conséquent de trouver les meilleures combinaisons possibles pour proposer un test simple et le plus sensible possible.

Une étude de sensibilité est en outre réalisée sur l'analyse de la co-expression de PD-1 et de CD38 à la surface des LT CD3 (CD3+) en association avec le ou les marqueurs : a) CD45RA (étude de la sous-population CD3+CD45RA-PD-1+CD38+) ; b) CD45RA, CD4 et CD8 (étude des sous-populations CD3+CD4+CD8-CD45RA-PD-1+CD38+ et CD3+CD4-CD8+CD45RA-PD-1+CD38+) ; c) CD45RA, CD4, CD8 et CD127 (étude des sous-populations CD3+CD4+CD8-CD45RA-CD127-PD-1+CD38+ et CD3+CD4-CD8+CD45RA-CD127-PD-1+CD38+) ; d) CD45RA, CD4, CD8, CD127 et CXCR5 (étude des sous-populations CD3+CD4+CD8-CD45RA-CD127-CXCR5-PD-1+CD38+ et CD3+CD4-CD8+CD45RA-CD127- CXCR5-PD-1+CD38+) ; et e) CD45RA, CD4, CD8, CD127, CXCR5 et CD27 (étude des sous-populations CD3+CD4+CD8-CD45RA-CD127-CXCR5-CD27+PD-1+CD38+ et CD3+CD4-CD8+CD45RA-CD127- CXCR5-CD27+PD-1+CD38+).

Cette étude consiste à identifier l'association de marqueur associé à la co-expression de PD-1 et de CD38 la plus sensible possible pour mesurer l'activité de l'auto-immunité chez les patients ayant une hépatite auto-immune.

### Listes des références

1. Liberal, R., Krawitt, E.L., Vierling, J.M., Manns, M.P., Mieli-Vergani, G., and Vergani, D. (2016). Cutting edge issues in autoimmune hepatitis. J. Autoimmun. 75, 6-19.
2. Manns, M.P., Czaja, A.J., Gorham, J.D., Krawitt, E.I., Mieli-Vergani, G., Vergani, D., Vierling, J.M., and American Association for the Study of Liver Diseases (2010). Diagnosis and management of autoimmune hepatitis. Hepatol. Baltim. Md 51, 2193-2213.
3. European Association for the Study of the Liver (2015). EASL Clinical Practice Guidelines: Autoimmune hepatitis. J. Hepatol. 63, 971-1004.
4. Renand, A., Habes, S., Mosnier, J.-F., Aublé, H., Judor, J.-P., Vince, N., Hulin, P., Nedellec, S., Métairie, S., Archambeaud, 1., et al. (2018). Immune Alterations in Patients With Type 1 Autoimmune Hepatitis Persist Upon Standard Immunosuppressive Treatment. Hepatol. Commun. 2, 968-981.
5. Böttcher, K., Rombouts, K., Saffioti, F., Roccarina, D., Rosselli, M., Hall, A., Luong, T., Tsochatzis, E.A., Thorburn, D., and Pinzani, M. (2018). MAIT cells are chronically activated in patients with autoimmune liver disease and promote pro-fibrogenic hepatic stellate cell activation. Hepatol. Baltim. Md.
6. Jeffery, H.C., van Wilgenburg, B., Kurioka, A., Parekh, K., Stirling, K., Roberts, S., Dutton, E.E., Hunter, S., Geh, D., Braitch, M.K., et al. (2016). Biliary epithelium and liver B cells exposed to bacteria activate intrahepatic MAIT cells through MRI. J. Hepatol. 64, 1118-1127.
7. Oo, Y.H., Banz, V., Kavanagh, O., Liaskou, E., Withers, D.R., Humphreys, E., Reynolds, G.M., Lee-Turner, L., Kalia, N., Hubscher, S.G., et al. (2012). CXCR3-dependent recruitment and CCR6-mediated positioning of Th-17 cells in the inflamed liver. J. Hepatol. 57, 1044-1051.

## Revendications

1. Procédé de prédiction et/ou de pronostic d'une hépatite auto-immune active chez un sujet, comprenant les étapes suivantes :
(i) détecter la co-expression de PD-1 (Programmed cell death 1) et CD38 à la surface des lymphocytes T, dans un échantillon de sang du sujet, et déterminer la fréquence de co-expression de PD-1 et CD38;
(ii) comparer la fréquence de co-expression de PD-1 et CD38 déterminée en (i) avec une valeur de référence de la fréquence de co-expression de PD-1 et de CD38 ;
(iii) rechercher la présence ou l'absence d'un écart de la fréquence de co-expression de PD-1 et de CD38 déterminée en (ii) par rapport à la valeur de référence ; et
(iv) attribuer la présence ou l'absence d'écart à une prédiction et/ou un pronostic de l'hépatite auto-immune active chez le sujet,
dans lequel une fréquence de co-expression de PD-1 et CD38, dans ledit échantillon, supérieure à une valeur de référence indique que le sujet présente un risque de rechute d'une hépatite auto-immune active.

2. Procédé selon la revendication 1, dans lequel ledit échantillon de sang est une fraction de sang ou un échantillon de sang total.

3. Procédé selon la revendication 1 ou 2, dans lequel les protéines PD-1 et CD38 sont détectées à la surface des lymphocytes T CD3 et/ou CD8 et/ou lymphocytes T CD4.

4. Procédé selon l'une quelconque des revendications précédentes, dans lequel on détecte l'expression d'au moins un autre marqueur biologique choisi dans le groupe consistant en CD3, CD4, CD8, CD45RA, CXCR5, CD127 et CD27.

5. Procédé selon la revendication 4 dans lequel on détecte la co-expression des associations de marqueurs biologiques choisis parmi les associations PD-1/CD38/CD3, PD-1/CD38/CD3/CD4/CD8/CD45RA, PD-1/CD38/CD3/CD4/CD8/CD45RA/CD127, PD-1/CD38/CD3/CD4/CD8/CD45RA/CXCR5/CD127 et PD-1/CD38/CD3/CD4/CD8/CD45RA/CXCR5/CD127/CD27.

6. Procédé selon la revendication 5, dans lequel on détecte la fréquence des populations lymphocytaires définis par les phénotypes ci-dessous:
a) CD3+CD45RA-PD-1+CD38+ ;
b) CD3+CD4+CD8-CD45RA-PD-1+CD38+ et/ou CD3+CD4-CD8+CD45RA-PD-1+CD38+ ;
c) CD3+CD4+CD8-CD45RA-CD127-PD-1+CD38+ et/ou CD3+CD4-CD8+CD45RA-CD127-PD-1+CD38+ ;
d) CD3+CD4+CD8-CD45RA-CD127-CXCR5-PD-1+CD38+ et/ou CD3+CD4-CD8+CD45RA-CD127- CXCR5-PD-1+CD38+ ; et
e) CD3+CD4+CD8-CD45RA-CD127-CXCR5-CD27+PD-1+CD38+ et/ou CD3+CD4-CD8+CD45RA-CD127 - CXCR5-CD27+PD-1+CD38+.

7. Procédé selon l'une quelconque des revendications précédentes, dans lequel la détection est réalisée par une méthode choisie parmi la cytométrie en flux ou de masse, une technologie de test immunologique, telle que ELISA direct, ELISA indirect, ELISA sandwich, ELISA compétitif, ELISA multiplex, un dosage radio-immunologique (RIA) ou une technologie ELISPOT, une méthode d'analyse par spectrométrie de masse, un procédé de chromatographie, procédé de qPCR et une combinaison d'au moins deux de ces méthodes.

8. Procédé selon l'une quelconque des revendications précédentes, dans lequel le-ladite prédiction est une prédiction du risque de rechute suite à l'arrêt ou à l'aménagement d'un traitement.

9. Procédé selon l'une quelconque des revendications précédentes, dans lequel ladite hépatite auto-immune est une hépatite auto-immune de forme atypique, notamment une forme séronégative et/ou avec peu ou pas d'infiltrat lymphocytaire du tissu hépatique.

10. Utilisation in vitro du pool de protéines PD-1 (Programmed cell death 1) et CD38 comme marqueurs biologiques co-exprimés à la surface des lymphocytes T, mesurés dans un échantillon de sang prélevé sur un sujet pour la prédiction et/ou le pronostic d'une hépatite auto-immune active chez un sujet.

## Patentansprüche

1. Verfahren zur Vorhersage und/oder Prognose von aktiver Autoimmunhepatitis bei einem Patienten, umfassend die folgenden Schritte:
(i) Nachweis der Koexpression von PD-1 (Programmed cell death 1) und CD38 auf der Oberfläche von T-Lymphozyten in einer Blutprobe des Probanden und Bestimmung der Häufigkeit der Koexpression von PD-1 und CD38;
(ii) die unter i) ermittelte Häufigkeit der Koexpression von PD-1 und CD38 mit einem Referenzwert für die Häufigkeit der Koexpression von PD-1 und CD38 vergleichen;
(iii) Suche nach dem Vorhandensein oder Nichtvorhandensein einer Abweichung der unter (ii) bestimmten Häufigkeit der Koexpression von PD-1 und CD38 vom Referenzwert; und
(iv) das Vorhandensein oder Nichtvorhandensein einer Abweichung mit der Vorhersage und/oder Prognose einer aktiven Autoimmunhepatitis bei dem Probanden in Verbindung bringen,
wobei eine Häufigkeit der Koexpression von PD-1 und CD38 in der Probe, die größer als ein Referenzwert ist, anzeigt, dass die Person ein Risiko für einen Rückfall der aktiven Autoimmunhepatitis hat.

2. Verfahren nach Anspruch 1, wobei die Blutprobe eine Blutfraktion oder eine Vollblutprobe ist.

3. Verfahren nach Anspruch 1 oder 2, wobei PD-1- und CD38-Proteine auf der Oberfläche von CD3- und/oder CD8- und/oder CD4-T-Lymphozyten nachgewiesen werden.

4. Verfahren nach einem der vorhergehenden Ansprüche, wobei die Expression von mindestens einem anderen biologischen Marker, ausgewählt aus der Gruppe bestehend aus CD3, CD4, CD8, CD45RA, CXCR5, CD127 und CD27, nachgewiesen wird.

5. Verfahren nach Anspruch 4, **dadurch gekennzeichnet, dass** die Koexpression von Assoziationen von biologischen Markern, ausgewählt aus den Assoziationen PD-1/CD38/CD3, PD-1/CD38/CD3/CD4/CD8/CD45RA, PD-1/CD38/CD3/CD4/CD8/CD45RA/CD127, PD-1/CD38/CD3/CD4/CD8/CD45RA/CXCR5/CD127 et PD-1/CD38/CD3/CD4/CD8/CD45RA/CXCR5/CD127/CD27.

6. Verfahren nach Anspruch 5, bei dem die Häufigkeit von Lymphozyten Populationen, die durch die nachstehenden Phänotypen definiert sind, erfasst wird:
a) CD3+CD45RA-PD-1+CD38+ ;
b) CD3+CD4+CD8-CD45RA-PD-1+CD38+ et/ou CD3+CD4-CD8+CD45RA-PD-1+CD38+ ;
c) CD3+CD4+CD8-CD45RA-CD127-PD-1+CD38+ et/ou CD3+CD4-CD8+CD45RA-CD127-PD-1+CD38+ ;
d) CD3+CD4+CD8-CD45RA-CD127-CXCR5-PD-1+CD38+ et/ou CD3+CD4-CD8+CD45RA-CD127- CXCR5-PD-1+CD38+ ; et
e) CD3+CD4+CD8-CD45RA-CD127-CXCR5-CD27+PD-1+CD38+ et/ou CD3+CD4-CD8+CD45RA-CD127- CXCR5-CD27+PD-1+CD38+.

7. Verfahren nach einem der vorhergehenden Ansprüche, wobei der Nachweis durch ein Verfahren, ausgewählt aus Durchfluss- oder Massenzytometrie, einer Immunoassay-Technologie, wie direktem ELISA, indirektem ELISA, Sandwich-ELISA, kompetitivem ELISA, Multiplex-ELISA, Radioimmunoassay (RIA) oder ELISPOT-Technologie, einem Massenspektrometrie-Analyseverfahren, einem Chromatographie verfahren, einem qPCR-Verfahren und einer Kombination von mindestens zwei dieser Verfahren, durchgeführt wird.

8. Verfahren nach einem der vorhergehenden Ansprüche, wobei die Vorhersage eine Vorhersage des Rückfallrisikos nach Abbruch oder Anpassung einer Behandlung ist.

9. Verfahren nach einem der vorhergehenden Ansprüche, wobei die Autoimmunhepatitis eine atypische Form der Autoimmunhepatitis ist, insbesondere eine seronegative Form und/oder mit wenig oder keinem Lymphozyten Infiltrat des Lebergewebes.

10. In vitro-Verwendung des PD-1- (Programmierter Zelltod 1) und des CD38-Proteinpools als biologische Marker, die auf der Oberfläche von T-Lymphozyten mit exprimiert werden, gemessen in einer von einem Probanden entnommenen Blutprobe, für die Vorhersage und/oder Prognose einer aktiven Autoimmunhepatitis bei einem Probanden.

## Claims

1. Method of predicting and/or prognosing active autoimmune hepatitis in a subject, comprising the following steps :
(i) detect the co-expression of PD-1 (Programmed cell death 1) and CD38 on the surface of T lymphocytes, in a blood sample of the subject, and determine the frequency of co-expression of PD-1 and CD38;
(ii) compare the frequency of co-expression of PD-1 and CD38 determined in (i) with a reference value for the frequency of co-expression of PD-1 and CD38;
(iii) search for presence or absence of a deviation of the frequency of co-expression of PD-1 and CD38 determined in (ii) from the reference value ; and
(iv) attribute the presence or absence of a deviation to prediction and/or a prognosis of active autoimmune hepatitis in the subject,
wherein a frequency of co-expression of PD-1 and CD38 in said sample greater than a reference value indicates that the subject is at risk of relapse of active autoimmune hepatitis.

2. Method according to claim 1, wherein said blood sample is a blood fraction or a whole blood sample.

3. Method according to claim 1 or 2, wherein PD-1 and CD38 proteins are detected on the surface of CD3 and/or CD8 and/or CD4 T lymphocytes.

4. Method according to any one of the preceding claims, wherein the expression of at least one other biological marker selected from the group consisting of CD3, CD4, CD8, CD45RA, CXCR5, CD127 and CD27 is detected.

5. Method according to claim 4, wherein is detected the co-expression of associations of biological markers chosen from the associations PD-1/CD38/CD3, PD-1/CD38/CD3/CD4/CD8/CD45RA, PD-1/CD38/CD3/CD4/CD8/CD45RA/CD127, PD-1/CD38/CD3/CD4/CD8/CD45RA/CXCR5/CD127 et PD-1/CD38/CD3/CD4/CD8/CD45RA/CXCR5/CD127/CD27.

6. Method according to claim 5, wherein is detected the frequency of lymphocytes populations defined by the phenotypes below:
a) CD3+CD45RA-PD-1+CD38+ ;
b) CD3+CD4+CD8-CD45RA-PD-1+CD38+ et/ou CD3+CD4-CD8+CD45RA-PD-1+CD38+ ;
c) CD3+CD4+CD8-CD45RA-CD127-PD-1+CD38+ et/ou CD3+CD4-CD8+CD45RA-CD127-PD-1+CD38+ ;
d) CD3+CD4+CD8-CD45RA-CD127-CXCR5-PD-1+CD38+ et/ou CD3+CD4-CD8+CD45RA-CD127 - CXCR5-PD-1+CD38+ ; et
e) CD3+CD4+CD8-CD45RA-CD127 -CXCR5-CD27 + PD-1 +CD38+ et/ou CD3+CD4-CD8+CD45RA-CD127- CXCR5-CD27+PD-1+CD38+.

7. Method according to any one of the preceding claims, wherein the detection is performed by a method selected from flow or mass cytometry, an immunoassay technology, such as direct ELISA, indirect ELISA, sandwich ELISA, competitive ELISA, multiplex ELISA, radioimmunoassay (RIA) or ELISPOT technology, a mass spectrometry analysis method, a chromatography method, a qPCR method and a combination of at least two of these methods.

8. Method according to any one of the preceding claims, wherein said prediction is a prediction of the risk of relapse following discontinuation or adjustment of a treatment.

9. Method according to any one of the preceding claims, wherein said autoimmune hepatitis is an atypical form of autoimmune hepatitis, in particular a seronegative form and/or with little or no lymphocytic infiltrate of the liver tissue.

10. In vitro use of the PD-1 (Programmed cell death 1) and CD38 protein pool as biological markers co-expressed on the surface of T lymphocytes, measured in a blood sample taken from a subject, for the prediction and/or prognosis of active autoimmune hepatitis in a subject.
